# EUROPÄISCHE PATENTSCHRIFT

(11) **EP 0 427 061 B1**
(45) Veröffentlichungstag und Bekanntmachung des Hinweises auf die Patenterteilung: **28.12.1994**
(21) Anmeldenummer: 90120548.4
(22) Anmeldetag: 26.10.1990
(51) Int. Cl.: C07D 405/06

(54) **Verfahren zur Herstellung von cis-2-(1H-1,2,4-Triazol-1-yl-methyl)-2-halogenphenyl)-3-(Halogenphenyl)-oxiran**
Process for the preparation of cis-2-(1H-1,2,4-triazol-1-yl-methyl)-2-(halophenyl)-3-(halophenyl)-oxirane
Procédé de préparation de cis-2-(1H-1,2,4-triazol-1-yl-méthyl)-2-(halophényl)-2-(halophényl)-3-oxirane

(30) Priorität: 04.11.1989 DE 3936821
(43) Veröffentlichungstag der Anmeldung: 15.05.1991
(73) Patentinhaber: BASF Aktiengesellschaft, 67063 Ludwigshafen (DE)
(72) Erfinder: Hickmann, Eckhard Dr., W-6701 Dannstadt 1 (DE); Seele, Rainer, Dr., W-6701 Fussgoenheim (DE); Kober, Reiner, Dr., W-6701 Fussgoenheim (DE); Isak, Heinz, Dr., W-6704 Mutterstadt (DE)

(56) Entgegenhaltungen:
- EP-A- 0 031 537
- EP-A- 0 196 038

## Beschreibung

Die vorliegende Erfindung betrifft ein Verfahren zur Herstellung von cis-2-(1H-1,2,4-Triazol-1-yl-methyl)-2-(halogenphenyl)-3-(halogenphenyl)-oxiran I 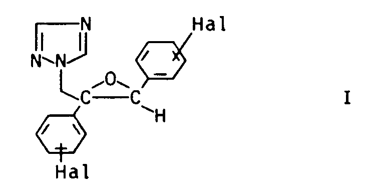
durch Epoxidation von Z-3-(1H-1,2,4-Triazol-1-yl)-2-(halogenphenyl)-1-(halogenphenyl)-propen II, 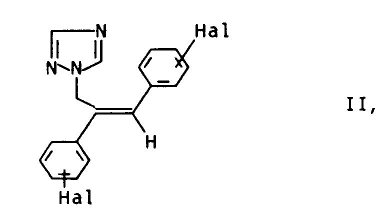
wobei Halogen jeweils Fluor, Chlor oder Brom bedeuten.

Die Herstellung von Epoxiden der Struktur I ist z.B. in DE-OS-32 18 129, 32 18 130 oder EP-A-196 038 beschrieben.

Üblicherweise werden nach einer Epoxidation eines Olefins mittels Peroxidverbindungen im rohen Reaktionsgemisch vorhandene Peroxidverbindungen wie Wasserstoffperoxid, Alkylhydroperoxide, Dialkylperoxide, Peroxicarbonsäuren oder Diacylperoxide, zerstört. Das gelingt z.B. durch katalytische Zersetzung an einem Edelmetallkontakt (üblicherweise Pt oder Pd) oder aber durch Zugabe von chemischen Reduktionsmitteln. Diese Nachbehandlung von Epoxidations-Rohprodukten hat rein sicherheitstechnische Gründe; man will damit sicherstellen, daß die durchweg energiereichen Peroxidverbindungen sich nicht mehr während der weiteren Aufarbeitung in unkontrollierter, verheerender Weise zersetzen können. Unter Peroxidverbindungen sind beispielsweise anorganische und organische Peroxide, Hydroperoxide und Persäuren zu verstehen.

Der Erfindung lag die Aufgabe zugrunde, die bislang nicht zufriedenstellenden Ausbeuten bei der Epoxidation der Olefine II und damit verbundenen hohen Reinigungsaufwand zu vermeiden.

Demgemäß wurde ein Verfahren zur Herstellung von cis-2-(1H-1,2,4-Triazol-1-yl-methyl)-2-(halogenphenyl)-3-(halogenphenyl)-oxiran I 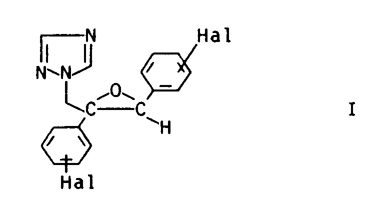
durch Epoxidation von Z-3-(1H-1,2,4-Triazol-1-yl)-2-(halogenphenyl)-1-(halogenphenyl)-propen II, 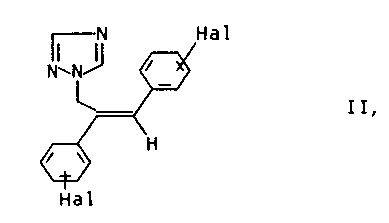
wobei Halogen jeweils Fluor, Chlor oder Brom bedeuten, mit Peroxidverbindungen gefunden, das dadurch gekennzeichnet ist, daß man nach der Epoxidation das Epoxidationsrohprodukt mit einem oder mehreren Reduktionsmitteln umsetzt, wobei das Reduktionsmittel dem Reaktionsgemisch in wesentlich höherer Menge zugesetzt wird, als für die Zerstörung von ggf. vorhandenen Peroxidverbindungen erforderlich ist.

Man beobachtet die überraschende Ausbeute- und Reinheits-steigernde Wirkung von Reduktionsmitteln bei dem vorliegenden, erfindungsgemäßen Verfahren nur, wenn man diese "überschüssig" einsetzt, d.h., mehr davon zugibt, als für die rasch verlaufende Reduktion von "überlebenden" Peroxidverbindungen, deren Menge man in üblicher Weise, z.B. durch iodometrische Titration bestimmen kann, notwendig ist. Arbeitet man dagegen die Reaktionsansätze nach der katalytischen oder reduktiven Zerstörung der restlichen Peroxidverbindungen ohne reduktive Nachbehandlung des peroxidfreien Reaktionsrohproduktes auf, so erhält man Ausbeuten von maximal 65 % der Theorie im Rohprodukt (siehe Vergleichsbeispiele). Durch Wertprodukt-Verluste bei den erforderlichen Reinigungsoperationen, z.B. beim Kristallisieren, sinkt die isolierte Ausbeute an Wertprodukt auf maximal 50% der Theorie.

Für die erfindungsgemäße reduktive Nachbehandlung kommt eine breite Auswahl von Reduktionsmitteln und -verfahren in Frage, z.B. katalytische Hydrierungen an Kontakten wie z.B. Pt, Pd, Cobalt oder Raney-Nickel,
Transfer-Hydrierungen mit z.B. Ammoniumformiat an Palladium,
Reduktionen mit Wasserstoff "in statu nascendi", z.B. Zink/Eisessig, Eisen/Salzsäure, Aluminium/Natronlauge,
Reduktionen mit komplexen Metallhydriden, z.B. Natriumborhydrid, Natriumdimethoxiborhydrid, Natriumtriacetoxiborhydrid,
Reduktion mit Element-Hydriden, z.B. Diboran,
Reduktion mit Salzen von Metallen in niedrigen Wertigkeitsstufen, z.B. Zinn(II)-chlorid, Eisen(II)-sulfat, Titan(III)-chlorid,
Reduktion mit Verbindungen von Elementen der 5. und 6. Hauptgruppe des Periodensystems in niedrigen Wertigkeitsstufen, z.B. Hydrazin, Hydroxylamin, Trimethylphosphit, Triphenylphosphoran, Phosphortrichlorid, Hydrophosphite, Thionylchlorid, Schwefeldioxid, Hydrogensulfite, Disulfite, Dithionite, Thiosulfate, Sulfinate, Hydrogensulfide, Sulfide, Alkali- oder Erdalkalihydrogensulfit, -sulfit, -disulfit oder dithionit,
Reduktion mit reduzierenden organischen Verbindungen z.B. mit Formaldehyd, Glyoxal, Glyoxylsäure, Ameisensäure, α-Hydroxisulfinsäuren, α-Hydroxisulfonsäuren und deren Salze, z.B. Alkali- oder Erdalkali-α-Hydroxialkylsulfonat oder -sulfinat.

Besonders bevorzugt sind reduktive Nachbehandlungen durch katalytische Hydrierung und mittels Sulfit, Hydrogensulfit, Disulfit oder Dithionit.

Das zusätzlich zur Reduktion von Restperoxid-Mengen überschüssig verwendete Reduktionsmittel wird in Anteilen von ca. 10 Mol-% bis ca. 2000 Mol-%, bevorzugt ca. 50 Mol-% bis ca. 1500 Mol-%, bezogen auf Z-3-(1H-1,2,4-Triazol-1-yl)-2-(halogenphenyl)-1-(halogenphenyl)-propen II, eingesetzt.

Die reduktive Nachbehandlung kann ein- oder mehrstufig erfolgen, z.B. kann man zuerst die restlichen Peroxidverbindungen reduzieren oder katalytisch am Edelmetallkontakt zersetzen, dann erst mit einem, ggf. anderen Reduktionsmittel in den oben angegebenen Überschußmengen nachbehandeln oder man kann beide Vorgänge in einem Zuge ausführen.

Die Behandlung mit überschüssigem Reduktionsmittel erfolgt zweckmäßigerweise in Gegenwart der zur Epoxidation verwendeten Lösungsmittel; das Reaktionsgemisch kann dabei ein- oder mehrphasig sein. Man kann aber auch, am besten nach Zerstören der restlichen Peroxidverbindungen, einen Lösungsmittelwechsel vornehmen, falls das Reduktionsmittel mit dem Lösungsmittel für die Epoxidation inkompatibel ist.

Als Lösungsmittel für die reduktive Nachbehandlung kommen z.B. in Frage: Aromatische Kohlenwasserstoffe, z.B. Benzol, Toluol, die Xylole; Ether, z.B. tert.-Butyl-methylether, Diethylenglykol-dimethylether; chlorierte Kohlenwasserstoffe, z.B. Methylenchlorid, 1,2-Dichlorethan, 1,1,1-Trichlorethan, Chlorbenzol; Alkohole, z.B. Methanol, iso-Propanol, sek.- und tert.-Butanol, Ethylenglykol; Carbonsäuren, z.B. Essigsäure und Propionsäure; Ester, z.B. Essigsäureethylester, Essigsäure-i-amylester, Buttersäuremethylester, Bernsteinsäure-dimethylester; Amide, z.B. Dimethylformamid, N-Methylpyrrolidon; Nitrile, z.B. Acetonitril, Harnstoffe, z.B. N,N,N′,N′-Tetramethylharnstoff, N,N′-Dimethyl-ethylen-und N,N′-Dimethylpropylen-harnstoff; Wasser, sowie ein- und mehrphasige Gemische der genannten Stoffe. Im allgemeinen wird man die Reduktionsmittel so auswählen, daß sie nicht schon selbst mit dem Lösungsmittel reagieren. Man kann sie aber auch bewußt so kombinieren, daß das wirksame Reduktionsmittel erst in situ gebildet wird, z.B. Diboran aus Natriumborhydrid und Methylenchlorid, Natriumdimethoxiboranat aus Natriumborhydrid und Methanol.

Die Reaktionstemperatur liegt bei der reduktiven Nachbehandlung in der Regel bei 0 bis 150°C, bevorzugt bei 20 bis 80°C.

Die Reaktionsdauer liegt im allgemeinen bei 0,5-10 Stunden, bevorzugt bei 1 bis 3 Stunden.

Bei der reduktiven Nachbehandlung hängt der bevorzugte pH vom bekannten Optimum des jeweiligen Reduktionsmittels ab. So verwendet man Natriumdithionit oder Natrium-Hydroximethylsulfinat im Alkalischen, Natriumhydrogensulfit im Sauren.

Das cis-2-(1H-1,2,4-Triazol-1-yl-methyl)-2-(halogenphenyl)-3-(halogenphenyl)-oxiran I wird nach an sich bekannten Verfahren isoliert: Durch Filtrieren oder Zentrifugieren oder, ggf. nach Phasentrennung, durch Ausfällen, Auskristallisieren oder Eindampfen. Zur Nachreinigung genügt in der Regel das Waschen oder Digerieren mit Wasser. Zur Herstellung von hochreinen Produkten kann man stattdessen oder zusätzlich mit einem organischen Lösungsmittel(-gemisch) digerieren oder daraus umkristallisieren.

Die Epoxidationsreaktion selbst wird in an sich bekannter Weise z.B. wie im eingangs zitierten Stand der Technik beschrieben, vorgenommen. Unter den dort angegebenen oder entsprechend abgewandelten Bedingungen oxidiert man die Olefine II mit Peroxycarbonsäuren wie Perbenzoesäure, 3-Chlorperbenzoesäure, 4-Nitroperbenzoesäure, Monoperphthalsäure, Peressigsäure, Perpropionsäure, Permaleinsäure, Monoperbernsteinsäure, Perpelargonsäure oder Trifluorperessigsäure in indifferenten Lösungsmitteln, vorzugsweise chlorierten Kohlenwasserstoffen, z.B. Methylenchlorid, Chloroform, Tetrachlorkohlenstoff, Dichlorethan, aber gegebenenfalls auch in Essigsäure, Essigester oder Dimethylformamid, gegebenenfalls in Gegenwart eines Puffers wie Natriumacetat, Natriumcarbonat, Natriumhydrogencarbonat, Dinatriumhydrogenphosphat. Man führt die Umsetzung bei Temperaturen zwischen 10 und 100°C aus und katalysiert die Reaktion gegebenenfalls mit Jod, Natriumwolframat oder Licht.

Bevorzugt kann man die Epoxidation in Gegenwart eines hohen Überschusses an Persäure, z.B. Permaleinsäure durchführen, die man vorteilhaft in situ aus 5 bis 30, insbesondere 5 bis 10 Moläquivalenten Maleinsäureanhydrid, bezogen auf das Olefin II, und weniger als stöchiometrische Mengen an Wasserstoffperoxidlösung, bezogen auf das Maleinsäureanhydrid herstellt. Im allgemeinen werden Molverhältnisse von Anhydrid zu H₂O₂ von 1,5 bis 10, insbesondere 2 bis 4 eingesetzt. Vorteilhaft kann eine 30 bis 50%ige wäßrige Lösung von Wasserstoffperoxid verwendet werden.

Die Reaktionstemperatur für die Epoxidierung kann 0 bis 100 °C, insbesondere 20 bis 80 °C betragen.

Die folgenden Beispiele veranschaulichen das erfindungsgemäße Verfahren, das sich auch auf andere Azolylmethyl-stilbene übertragen läßt:

### Beispiel 1

Man löst bei 40°C 58,9 g (0,601 mol) Maleinsäureanhydrid und 18,6 g 97,2 %iges (0,0576 mol) Z-3-(1H-1,2,4-Triazol-1-yl-methyl)-2-(fluorphenyl)-1-(2-chlorphenyl)-propen in 150 ml 1,2-Dichlorethan, tropft innerhalb von 1 h 20,6 g (0,303 mol) 50 %iges wäßriges Wasserstoffperoxid gleichmäßig zu und rührt das Reaktionsgemisch 7 h bei 40°C. Eine unter Rühren entnommene Probe weist bei der iodometrischen Titration 0,4 % Peroxidverbindungen, berechnet als Wasserstoffperoxid, auf; das erfordert 6,9 ml 38 %ige Natriumhydrogensulfit-Lösung zur Reduktion der Peroxidverbindungen. Nach Zugabe von 100 ml Wasser und Abpuffern der wäßrigen Phase auf pH 3 mit 50%iger Natronlauge setzt man 170 ml 38 %ige Natriumhydrogensulfitlösung zu, entsprechend einem Überschuß von 1385 Mol%, bezogen auf das eingesetzte Olefin und rührt das zweiphasige Reaktionsgemisch intensiv 3 h bei 50°C. Nach Neutralisieren der wäßrigen Phase mit 50 %iger Natronlauge gewinnt man ca. 94 % des eingesetzten 1,2-Dichlorethans durch Azeotropdestillation bei Normaldruck zurück (Azeotrop-Kp.: 72°C). Das beim Abkühlen der zurückbleibenden wäßrigen Phase vollständig auskristallisierende cis-2-(1H-1,2,4-Triazol-1-yl-methyl)-2-(4-fluorphenyl)-3-(2-chlorphenyl)-oxiran wird abgetrennt, gründlich mit Wasser gewaschen und bei 90°C im Vakuum getrocknet. Man erhält 17,7 g Produkt mit einem Gehalt von 93,4 %, bestimmt durch quantitative Hochdruckflüssigkeitschromatographie (HPLC). Das entspricht einer Ausbeute von 87 % der Theorie.

### Beispiel 2

Man führt die Epoxidation wie in Beispiel 1 beschrieben durch, filtriert die nach dem Abkühlen weitgehend ausgefallene Maleinsäure ab, wäscht den Filterkuchen mit wenig 1,2-Dichlorethan nach und vereinigt Filtrat und Waschflüssigkeit. Diese Rohproduktlösung wiegt 242 g und enthält nach iodometrischer Titration 0,08 % Peroxidverbindungen, berechnet als Wasserstoffperoxid. Das erfordert 0,99 g Natriumdithionit zur Reduktion der Perverbindungen. Nach Zugabe von 12,0 g Natriumdithionit in 40 ml Wasser, entsprechend einem Überschuß von 110 Mol%, bezogen auf eingesetztes Olefin, rührt man das zweiphasige Reaktionsgemisch 3 h bei 70 bis 75°C und arbeitet die abgetrennte organische Phase auf. Man erhält 18,0 g cis-2-(1H-1,2,4-Triazol-1-yl-methyl)-2-(4-fluorphenyl)-3-(2-chlorphenyl)-oxiran mit einem Gehalt von 95,2% (quant. HPLC), entsprechend einer Ausbeute von 90,2 % der Theorie.

### Beispiel 3

In Analogie zu Beispiel 1 rührt man das Gemisch aus 9,3 g 97,2%igem (0,0288 mol) Z-3-(1H-1,2,4-Triazol-1-yl-methyl)-2-(4-fluorphenyl)-1-(2-chlorphenyl)-propen, 55 ml 1,2-Dichlorethan, 28 g Maleinsäureanhydrid, 0,3 g 2,6-Di-tert.-butylphenol und 7,3 g 50%igem Wasserstoffperoxid 6 h bei 50°C und weitere 2 h bei 70°C. Nach Zerstören der geringen Reste an Peroxidverbindungen durch Zugabe von 10 %iger wäßriger Natriumthiosulfatlösung, Neutralisieren des Reaktionsgemisches mit 50 %iger Natronlauge, Abtrennen und Eindampfen der organischen Phase löst man den Rückstand in 70 ml Methanol, gibt 0,5 g Raney-Nickel zu und rührt 2 h bei 50°C unter 2 l Wasserstoff (entsprechend 310 Mol%, bezogen auf eingesetztes Olefin). Nach Abfiltrieren des Katalysators und Eindampfen der Lösung erhält man 8,9 g cis-2-(1H-1,2,4-Triazol-1-yl-methyl)-2-(4-fluorphenyl)-3-(2-chlorphenyl)-oxiran mit einem Gehalt von 94,1 % (quant. HPLC), entsprechend einer Ausbeute von 88,2 % der Theorie.

### Vergleichsbeispiele

### Vergleichsbeispiel 1

Man führt die Epoxidation wie in Beispiel 1 beschrieben durch, reduziert restliche Peroxidverbindungen mit der gerade ausreichenden Menge an 38%iger Natriumhydrogensulfitlösung (8,2 ml) und arbeitet ohne reduktion Nachbehandlung mit überschüssiger Bisulfitlösung auf. Man erhält 17,2 g Rohprodukt mit einem Gehalt von 70,4% cis-2-(1H-1,2,4-Triazol-1-yl-methyl)-2-(4-fluorphenyl)-3-(2-chlorphenyl)-oxiran (quant. HPLC), entsprechend einer Ausbeute von 63,7% der Theorie.

### Vergleichsbeispiel 2

Bei gleicher Durchführung der Epoxidation wie bei Beispiel 2, Zerstören restlicher Peroxiverbindungen mit der gerade ausreichenden Menge an Natriumdithionit (1,4 g), aber ohne reduktive Nachbehandlung mit überschüssiger Dithionitlösung erhält man 17,5 g Rohprodukt mit einem Gehalt von 66,7% cis-2-(1H-1,2,4-Triazol-1-yl-methyl)-2-(4-fluorphenyl)-3-(2-chlorphenyl)-oxiran, entsprechend einer Ausbeute von 61,4% der Theorie.

### Vergleichsbeispiel 3

Man verfährt wie in Beispiel 3 beschrieben, unterläßt jedoch die reduktive Nachbehandlung des peroxidfreien Rohproduktes mit Wasserstoff und Raney-Nickel. Man erhält 8,6 g Rohprodukt mit einem Gehalt von 65,6% cis-2-(1H-1,2,4-Triazol-1-yl-methyl)-2-(4-fluorphenyl)-3-(2-chlorphenyl)-oxiran, entsprechend einer Ausbeute von 59,4% der Theorie.

## Patentansprüche

1. Verfahren zur Herstellung von cis-2-(1H-1,2,4-Triazol-1-yl-methyl)-2-(halogenphenyl)-3-(halogenphenyl)-oxiran I 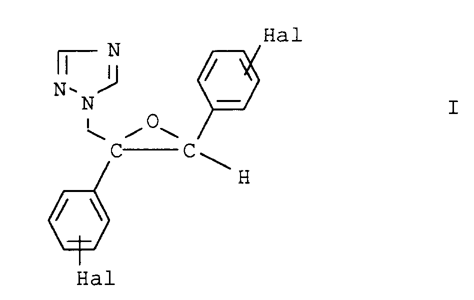 durch Epoxidation von Z-3-(1H-1,2,4-Triazol-1-yl)-2-(halogenphenyl)-1-(halogenphenyl)-propen II, 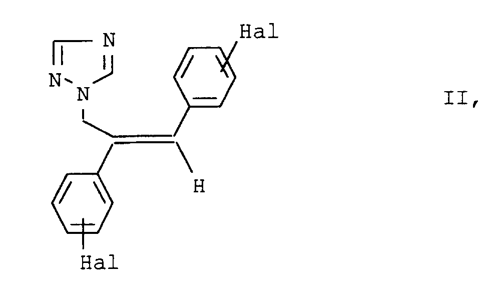 wobei Halogen jeweils Fluor, Chlor oder Brom bedeuten, mit Peroxidverbindungen, dadurch gekennzeichnet, daß man nach der Epoxidation das Epoxidationsrohprodukt mit einem oder mehreren Reduktionsmitteln umsetzt, wobei das Reduktionsmittel dem Reaktionsgemisch in wesentlich höherer Menge zugesetzt wird, als für die Zerstörung von ggf. vorhandenen Peroxidverbindungen erforderlich ist, wobei nach der Zerstörung der Peroxidverbindungen die Menge an verwendeten Reduktionsmittel(n) 10-2000 Mol-%, bezogen auf eingesetztes Z-3-(1H-1,2,4-Triazol-1-yl)-2-(halogenphenyl)-1-(halogenphenyl)-propen, beträgt.

2. Verfahren nach Anspruch 1, dadurch gekennzeichnet, daß man das peroxidhaltige Rohprodukt zunächst von Peroxidverbindungen befreit und das peroxidfreie Epoxidationsrohprodukt anschließend reduktiv nachbehandelt.

3. Verfahren nach Anspruch 1, dadurch gekennzeichnet, daß man die reduktive Nachbehandlung mit Wasserstoff oder einem Wasserstoffdonator und einem metallischen Katalysator aus der Gruppe der Schwermetalle Nickel, Cobalt, Platin oder Palladium vornimmt.

4. Verfahren nach Anspruch 1, dadurch gekennzeichnet, daß man die reduktive Nachbehandlung mit einem Alkali- oder Erdalkalihydrogensulfit, -sulfit, -disulfit oder -dithionit vornimmt.

5. Verfahren nach Anspruch 1, dadurch gekennzeichnet, daß man die reduktive Nachbehandlung bei Temperaturen von 20 bis 80°C vornimmt.

6. Verfahren nach Anspruch 1, dadurch gekennzeichnet, daß man cis-2-(1H-1,2,4-Triazol-1-yl-methyl)-2-(4-fluorphenyl)-3-(2-chlorphenyl)-oxiran herstellt.

## Claims

1. A process for the preparation of cis-2-(1H-1,2,4-triazol-1-ylmethyl)-2-(halophenyl)-3-(halophenyl)oxirane I 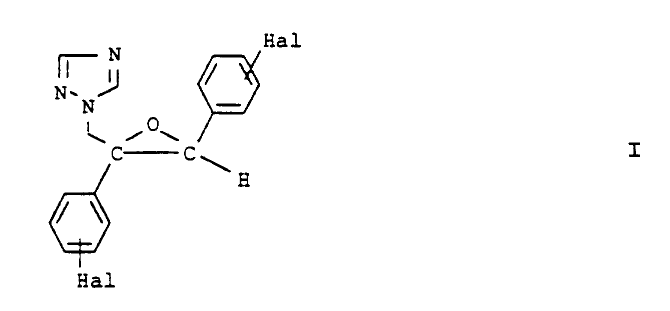 by epoxidizing Z-3-(1H-1,2,4-triazol-1-yl)-2-(halophenyl)-1-(halophenyl)propene II, 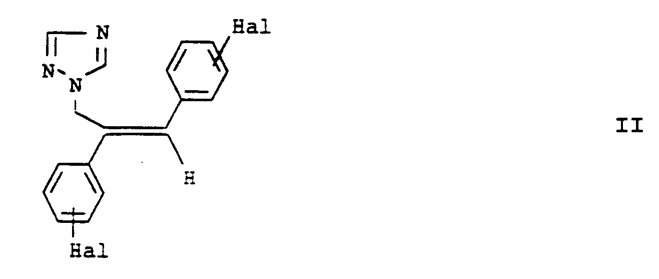 where halogen is in each case fluorine, chlorine or bromine, using a peroxide compound, which comprises reacting the crude product of the epoxidation with one or more reducing agents, which are added to the reaction mixture in a considerable excess over the amount necessary to destroy any peroxide compounds present, wherein, after the destruction of the peroxide compounds, the amount of reducing agent(s) used is from 10 to 2000 mol-%, based on the Z-3-(1H-1,2,4-triazol-1-yl)-2-(halophenyl)-1-(halophenyl)propene employed.

2. A process as claimed in claim 1, wherein the peroxide-containing crude product is first freed from peroxide compounds, and the peroxide-free crude product of the epoxidation is subsequently subjected to reductive aftertreatment.

3. A process as claimed in claim 1, wherein the reductive aftertreatment is carried out using hydrogen or a hydrogen donor and a metallic catalyst from the group comprising the heavy metals nickel, cobalt, platinum and palladium.

4. A process as claimed in claim 1, wherein the reductive aftertreatment is carried out using a hydrogen sulfite, sulfite, disulfite or dithionite of an alkali metal or alkaline earth metal.

5. A process as claimed in claim 1, wherein the reductive aftertreatment is carried out at from 20 to 80°C.

6. A process as claimed in claim 1, wherein cis-2-(1H-1,2,4-triazol-1-ylmethyl)-2-(4-fluorophenyl)-3-(2-chlorophenyl)oxirane is prepared.

## Revendications

1. Procédé de préparation de cis-2-[1H-1,2,4-triazol-1-yl-méthyl)-2-(halogénphényl)-3-(halogénphényl)-oxiranne I 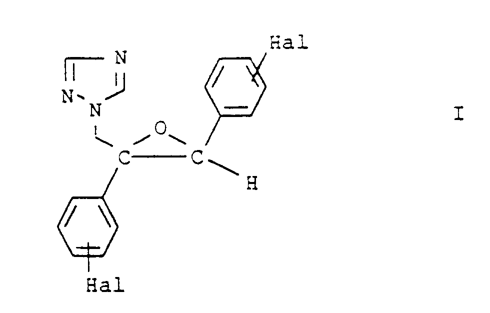 par époxydation de Z-3-(1H-1,2,4-triazol-1-yl)-2-(halogénophényl)-1-(halogénophényl)-propène II, 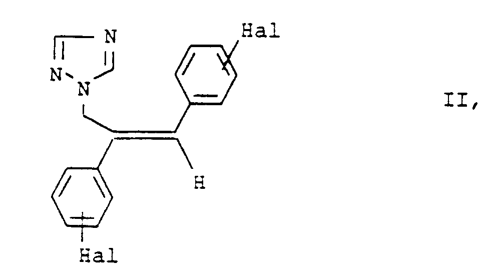 où halogène représente fluor, chlore ou brome, avec des composés péroxydiques, caractérisé par le fait que, après époxydation, on fait réagir le produit d'époxydation brut avec un ou plusieurs agents de réduction, l'agent de réduction étant ajouté au mélange de réaction en quantité sensiblement plus élevée que celle nécessaire pour la destruction des composés péroxydiques éventuellement présents, la quantité d'agents de réduction utilisés, après la destruction des composés péroxydiques, s élevant à 10-2000 % mol, rapportés au Z-3-(1H-1,2,4-triazol-1-yl)-2-(halogénophényl)-1-(halogénophényl)-propène introduit.

2. Procédé selon la revendication 1, caractérisé par le fait que l'on débarrasse d'abord de composés péroxydiques le produit brut à teneur en péroxyde et l'on traite ensuite par réduction le produit brut d'époxydation exempt de péroxyde.

3. Procédé selon la revendication 1, caractérisé par le fait que l'on effectue le traitement ultérieur par réduction avec de l'hydrogène ou un donneur d'hydrogène et un catalyseur métallique du groupe des métaux lourds, nickel, cobalt, platine ou palladium.

4. Procédé selon la revendication 1, caractérisé par le fait que l'on effectue le traitement ultérieur par réduction avec de l'hydrogénosulfite, sulfite, disulfite ou dithionite alcalin ou alcalino-terreux.

5. Procédé selon la revendication 1, caractérisé par le fait que l'on effectue le traitement ultérieur par réduction à des températures de 20 à 80°C.

6. Procédé selon la revendication 1, caractérisé par le fait que l'on prépare du cis-2-(1H-1,2,4-triazol-1-yl-méthyl)-2-(4-fluor-phényl)-3-(2-chlorophényl)-oxiranne.
